# EUROPEAN PATENT APPLICATION

(11) **EP 4 470 595 A2**
(43) Date of publication of application: **04.12.2024**
(21) Application number: 24193152.6
(22) Date of filing: 23.01.2017
(51) Int. Cl.: A61M 37/00

(54) **DELIVERY OF BOTULINUM WITH MICRONEEDLE ARRAYS**

(30) Priority: 22.01.2016 US 201662286255 P; 21.10.2016 US 201615331469
(62) Divisional of application: 17742133.6
(71) Applicant: Transderm, Inc., North Chicago, IL 60064 (US)
(72) Inventor: KASPAR, Roger L., Santa Cruz, CA, 95060 (US); SPEAKER, Tycho, Santa Cruz, CA, 95060 (US)
(74) Representative: Simmons & Simmons LLP (Munich)

(57) **Abstract**

A therapeutic toxin dosage form for treating a disease, disorder, or condition in a subject can include a microneedle array and a therapeutically effective amount of a therapeutic toxin loaded to the microneedle array for administration to the subject. Additionally, a method of treating a disease, disorder, or condition in a subject can include administering a therapeutically effective amount of a therapeutic toxin to the subject via a microneedle array.

## Description

### PRIORITY DATA

This application claims the benefit of United States Patent Application Serial No. 15/331,469 filed October 21, 2016 and United States Provisional Patent Application Serial No. 62/286,255 filed January 22, 2016, each of which is incorporated herein by reference.

### BACKGROUND

A variety of skin conditions result from dominant mutations in the keratins (K) such as pachyonychia congenita (PC), which is caused by mutations in the inducible keratins K6a, K6b, K6c, K16 and K17. While disabling painful plantar keratoderma is commonly accepted as the primary symptom affecting patient quality of life in PC patients, hyperhidrosis appears to contribute to blister formation and pain. In some cases, multiple intradermal injections of botulinum toxin (BTX) can effectively treat hyperhidrosis and substantially reduce pain and blistering. However, the number of dermal injections associated with this treatment protocol makes this procedure costly and difficult for the patient. For example, BTX intradermal injection is generally only feasible with pain control through tourniquet intravenous regional (IVR) or general anesthesia. Further, injections are typically spaced on approximately a 1.5 cm grid, translating to roughly 50-150 injections per foot, and symptomatic relief lasts as few as 1.5-6 months, making the treatment burden of regular maintenance injections substantial.

### BRIEF DESCRIPTION OF THE DRAWINGS

FIG. 1A depicts micrographs showing sweat duct distribution in age and gender matched PC (pachyonychia congenita)-affected vs healthy control volunteers in matched plantar regions. Stitched composites were produced using Adobe Photoshop CS2. Images were acquired using a Caliber ID VivaScope 1500.
FIG. 1B depicts a magnified image of palmar sweat gland in a non-PC volunteer (VivaStack rendered as 3D-structure with Amira 5.2.0).
FIG. 2A illustrates how polymer needle structures can penetrate into human skin to a typical depth of 50-100 microns, where the embedded tips rapidly hydrate in the moist epidermal and dermal environment to form transient depots.
FIG. 2B illustrates the tip of a 26-gauge hypodermic needle adjacent to an example dissolvable microneedle alternative. Beveled tips and compound curves aid thick skin penetration.
FIG. 2C illustrates a scanning electron micrograph of loaded protrusions of a microneedle structure before skin administration.
FIG. 2D illustrates a scanning electron micrograph of loaded protrusions of a microneedle structure after skin administration.
FIG. 3A shows a schematic of one embodiment of a method of fabrication of a microneedle array where a pin template (top, left panel) and glass slide (bottom, left panel) are covered with a thin film of viscous (20%) polyvinyl alcohol (PVA) solution. The pin template is placed in contact with the PVA solution (middle panel). Microneedles are produced by withdrawing the pins as the film is drying, forming fiber-like structures (right panel).
FIG. 3B illustrates an enlarged view of fibers depicted in FIG. 3A.
FIG. 3C depicts protrusions that are subsequently trimmed to the desired length and tip shape or bevel.
FIG. 3D illustrates a microneedle array supported by a glass substrate, with a penny to show scale.
FIG. 3E depicts a micrograph of one microneedle after trimming to 1 mm length, showing beveled structure that facilitates skin penetration, with a human hair (∼100 µm diameter) to show scale.
FIG. 3F illustrates microneedle arrays loaded alternatively with fluorescein or R-phycoerythrin.
FIG. 4A depicts a film in contact with iodine-treated human palmar skin showing dark spots over sweat pores.
FIG. 4B depicts a PVA film after removal from skin, with some light generalized staining visible, but showing distinct punctate dark spots corresponding to sweat pores.
FIG. 4C illustrates an example of threshold detection of active glands for quantitative measurement.
FIG. 4D depicts a graph showing water intensity response of a paper based starch-iodine film scaled to pore size, indicating that approximately 8-10 pL of water produces a measurable response.
FIG. 5A illustrates a footprint showing capability of this approach to record sweat production over large areas (e.g., entire sole) in a single operation.
FIG. 5B illustrates a close-up view of detail recorded in the film, with individual pores readily discernable within skin ridges of plantar surface. Not all pores are active in this healthy volunteer, and light areas correspond to lack of contact of PVA film and dry sweat duct openings. Ridges are visible due to moisture content, although these are readily discerned versus the more intense coloration of the pores. Scale bar = 500µm.
FIG. 6 illustrates two starch-containing films coupled together with an underlying strip of adhesive film that was affixed before moisture exposure. The film area overlying the adhesive film demonstrates increased sensitivity in detecting moisture content deposited by contact with a skin surface.
FIG. 7A illustrates films exposed to negative control footpads without treatment showing equivalent staining intensity on each foot.
FIG. 7B illustrates films exposed to footpads visualized 24 h following intradermal injection of saline negative control (left) and 50 units BTA in saline (right). BTA-treated paw exhibits lighter staining, consistent with reduction of sweat production.
FIG. 7C illustrates films exposed to footpads analyzed 4 days following treatment with blank microneedle array (left foot) application and BTA-loaded microneedle array (delivering approximately 50 units BTA, right foot). The paw (right) receiving BTA delivered by the microneedle array shows lighter staining consistent with BTA-mediated reduction of sweat production.
FIG. 8A illustrates a Digital Abduction Scale (DAS) showing increasing levels of paralysis at each DAS value.
FIG. 8B illustrates a photograph of a mouse with a DAS=4 response following Flex-PAD BTX administration (right hind limb) and DAS=0 response following Flex-PAD control administration (left hind limb).
FIG. 8C depicts a bar graph showing DAS readouts from mice treated with Flex-PADs containing BTX and intramuscular injection of BTX. Legend shows dose in ng.

Reference will now be made to the exemplary embodiments illustrated, and specific language will be used herein to describe the same. It will nevertheless be understood that no limitation of the scope of the invention is thereby intended.

### DESCRIPTION OF EXAMPLE EMBODIMENT(S)

Before particular embodiments of the present invention are disclosed and described, it is to be understood that this invention is not limited to the particular process and materials disclosed herein as such may vary to some degree. It is also to be understood that the terminology used herein is used for describing particular embodiments only and is not intended to be limiting.

As used in this written description, the singular forms "a," "an," and "the" shall include express support for plural referents unless the context clearly dictates otherwise. Thus, for example, reference to "a microneedle" includes reference to one or more microneedles, and reference to "the polymer" includes reference to one or more materials.

As used herein, the term "about" is used to provide flexibility to a numerical range endpoint by providing that a given value may be "a little above" or "a little below" the endpoint.

The term "subject" refers to a mammal that may benefit from the administration using a transdermal device or method of this invention. Examples of subjects include humans, and other animals such as horses, pigs, cattle, dogs, cats, rabbits, and aquatic mammals.

As used herein, the term "active agent" or "drug" are used interchangeably and refer to a pharmacologically active substance or composition. In one embodiment, an active agent can comprise or consist of a botulinum toxin.

As used herein, an "external longitudinal channel" is defined as being a channel or groove that runs along at least a portion of the longitudinal axis of the microneedle and which is open to the outside or exterior of the microneedle along at least a portion of its length.

As used herein, the term "substantially" refers to the complete or nearly complete extent or degree of an action, characteristic, property, state, structure, item, or result.

As used herein, sequences, compounds, formulations, delivery mechanisms, or other items may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

As used herein, the term "toxin" is used to mean any useful poison, including those from biological and non-biological sources, and further describes any agent that produces a disturbance or impairment in a biological system. Such agents may be harmful to organisms in sufficiently high doses, but yet may produce a useful therapeutic effect when delivered at a preselected dose. In some examples, toxins may specifically refer to small molecules, peptides, or proteins that produce a disruptive or inhibitory effect upon at least one type of cell in an organism, and may thereby induce a desirable or useful resultant change in the organism. The term "biotoxin" may further specify toxins of biological origin, which frequently target specific organelles or cellular structures such as ion-channels or receptors, or enzymatically or non-enzymatically cleave specific proteins such that specific cellular functions are disrupted.

As used herein, the term "botulinum toxin" (BTX) refers to a family of related, acutely neurotoxic proteins produced by the bacterium Clostridium botulinum and related species, comprising two protein chains, a 100-kDa heavy chain polypeptide and a 50-kDa light chain polypeptide joined by a disulfide linkage. Seven serologically distinct toxin types exist, exhibiting significantly different sequences and tertiary structures and are designated types A to G, which act in similar manners but can show different speciesspecific potency and pharmacodynamics/pharmacokinetics.

As used herein, a "derivative" is a compound obtained from a source compound, an analog, homolog tautomeric form, stereoisomer, polymorph, hydrate, pharmaceutically acceptable salt or pharmaceutically acceptable solvate thereof, by a simple chemical process converting one or more functional groups, by means of oxidation, hydrogenation, alkylation, esterification, halogenation and the like. The term "analog" refers to a compound having a structure similar to that of another one, but differing from it with respect to a certain component. The compound may differ in one or more atoms, functional groups, or substructures, which may be replaced with other atoms, groups, or substructures. In one aspect, such structures possess at least the same or a similar therapeutic efficacy for a given indication. The term "tautomer" or "tautomeric form" refers to structural isomers of different energies which are interconvertible via a low energy barrier. The term "stereoisomer" refers to one of a set of isomers whose molecules have the same number and kind of atoms bonded to each other, but which differ in the way these atoms are arranged in space. The term "polymorph" refers to crystallographically-distinct forms of a substance. In addition, an agent can be said to be "derived" from a source containing many compounds or agents, such as a plant, fungus, bacteria, or other organism. In this context, the agent can be described or otherwise referred to in terms of its source, rather than by its own properties, characteristics, name, or attributes per se. For example, an extract obtained from a plant may be described as "derived" from the plant. Further, in some examples, the derivative can be more or less potent than the source compound and/or can be more or less toxic than the source compound. In some specific examples, the derivative can be less potent and/or less toxic than the source compound.

The terms "treat," "treating," or "treatment" as used herein and as well understood in the art, mean an approach for obtaining beneficial or desired results, including without limitation clinical results in a subject being treated. Beneficial or desired results can include, but are not limited to, alleviation or amelioration of one or more signs or symptoms of a condition, diminishment of extent of disease, stabilizing (i.e. not worsening) the state of a disease or condition, delaying or slowing of disease progression, amelioration or palliation of the disease state, diminishment of the reoccurrence of disease, and remission (whether partial or total), whether detectable or undetectable. "Treat," "treating" and "treatment" can also mean prolonging survival as compared to expected survival if not receiving treatment and can be prophylactic. Such prophylactic treatment can also be referred to as prevention or prophylaxis of a disease or condition. The prophylaxis may be partial or complete. Partial prophylaxis may result in the delayed onset of a physiological condition.

As used herein, "comprises," "comprising," "containing" and "having" and the like can have the meaning ascribed to them in U.S. Patent law and can mean "includes," "including," and the like, and are generally interpreted to be open ended terms. The terms "consisting of' or "consists of' are closed terms, and include only the components, structures, steps, or the like specifically listed in conjunction with such terms, as well as that which is in accordance with U.S. Patent law. "Consisting essentially of'' or "consists essentially of'' have the meaning generally ascribed to them by U.S. Patent law. In particular, such terms are generally closed terms, with the exception of allowing inclusion of additional items, materials, components, steps, or elements, that do not materially affect the basic and novel characteristics or function of the item(s) used in connection therewith. For example, trace elements present in a composition, but not affecting the composition's nature or characteristics would be permissible if present under the "consisting essentially of' language, even though not expressly recited in a list of items following such terminology. When using an open ended term in the written description, like "comprising" or "including," it is understood that direct support should be afforded also to "consisting essentially of'' language as well as "consisting of' language as if stated explicitly and vice versa.

The term "dosage unit" or "dose" are understood to mean an amount of an active agent that is suitable for administration to a subject in order achieve or otherwise contribute to a therapeutic effect. In some embodiments, a dosage unit can refer to a single dose which is capable of being administered to a subject or patient, and that may be readily handled and packed, remaining as a physically and chemically stable unit dose.

The phrase "effective amount," "therapeutically effective amount," or "therapeutically effective rate(s)" of an active ingredient refers to a non-toxic, but sufficient amount or delivery rates of the active ingredient, to achieve therapeutic results in treating a disease or condition for which the drug is being delivered. It is understood that various biological factors may affect the ability of a substance to perform its intended task. Therefore, an "effective amount," "therapeutically effective amount," or "therapeutically effective rate(s)" may be dependent in some instances on such biological factors. Further, while the achievement of therapeutic effects may be measured by a physician or other qualified medical personnel using evaluations known in the art, it is recognized that individual variation and response to treatments may make the achievement of therapeutic effects a subjective decision. The determination of a therapeutically effective amount or delivery rate is well within the ordinary skill in the art of pharmaceutical sciences and medicine.

The term "extract" refers to those substances prepared using a solvent, e.g., ethanol, water, steam, superheated water, methanol, hexane, chloroform liquid, liquid CO2, liquid N2, propane, supercritical CO2, the like, or any combination thereof. Extracts, as used herein, can refer to an extract in a liquid form, or can refer to a product obtained from further processing of the liquid form, such as a dried powder or other solid form. Extracts may take many forms including but not limited to: solid, liquid, particulate, chopped, distillate, etc. and may be performed by any number of procedures or protocols, such as chopping, grinding, pulverizing, boiling, steaming, soaking, steeping, infusing, applying a gas, etc., and may employ any suitable reagents, such as water, alcohol, steam, or other organic materials. Extracts typically have a given purity percentage and can be relatively to highly pure. In some embodiments, extracts can be phytoextracts made from specific parts of a source, such as the skin, pulp, leaves, flowers, fruits of a plant etc., or can be made from the whole source. In some aspects an extract may include one or more active fractions or active agents. In some extracts, additional ingredients can be added as a carrier. In some aspects, the purity and/or activity of an extract can be controlled by, or be a function of the specific bacterial source strain, the method of cultivation, the extraction process, or the protocol followed in producing the extract. Methods for producing, extracting, and purifying specific botulinum toxins are known and described in the literature, such as in US Patent 7189541, which is incorporated herein by reference. For example the well-established Schantz process can be used to obtain crystalline botulinum toxin type A, described in Schantz, E. J., et al, Properties and use of Botulinum toxin and Other Microbial Neurotoxins in Medicine, Microbiol Rev. 56: 80-99 (1992). A typical method entails anaerobic fermentation of a suitable strain followed by lysis and filtration of the culture, and precipitation of the toxin with sulfuric acid. Dissolution of the precipitate in a calcium chloride solution and reprecipitation with ethanol, followed by resuspension in phosphate buffer and drying of the centrifuged supernatant to produce high-potency crystalline botulinum toxin type A. Adaptations of this and other suitable methods may be used to produce purified extracts of other therapeutic toxins.

As used herein, "formulation" and "composition" can be used interchangeably and refer to a combination of at least two ingredients. In some embodiments, at least one ingredient may be an active agent or otherwise have properties that exert physiologic activity when administered to a subject.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 0.5 to 10 g" should be interpreted to include not only the explicitly recited values of about 0.5 g to about 10.0 g, but also include individual values and sub-ranges within the indicated range. Thus, included in this numerical range are individual values such as 2, 5, and 7, and sub-ranges such as from 2 to 8, 4 to 6, etc. This same principle applies to ranges reciting only one numerical value. Furthermore, such an interpretation should apply regardless of the breadth of the range or the characteristics being described.

Unless otherwise defined, all technical and scientific terms used herein have the same meaning as commonly understood to one of ordinary skill in the art to which this invention belongs. Although any methods, devices and materials similar or equivalent to those described herein can be used in the practice or testing of the invention, representative methods, devices, and materials are described below.

It is noted that when discussing the devices, systems and associated methods, each of these discussions can be considered applicable to each of these embodiments, whether or not they are explicitly discussed in the context of that embodiment. Thus, for example, in discussing a therapeutic toxin dosage form, such a dosage form can also be used in a method of treating a disease, disorder, or condition, and vice versa.

As a general overview, a variety of skin conditions, and pachyonychia congenita (PC) in particular, can result from dominant mutations in the inducible keratins (K) including K6a, K6b, K16 and K17. While disabling painful plantar keratoderma is commonly accepted as the primary symptom affecting patient quality of life in PC patients, hyperhidrosis appears to contribute to blister formation and pain. In some cases, multiple intradermal injections of the therapeutic toxin known as botulinum toxin (BTX) can effectively treat hyperhidrosis and substantially reduce pain and blistering. However, the number of dermal injections associated with this treatment protocol makes this procedure costly and difficult on the patient (regional nerve blocks or general anesthesia are used). For example, BTX intradermal injection is generally only feasible with pain control through tourniquet intravenous regional (IVR) or general anesthesia. Further, injections are typically spaced on approximately a 1.5 cm grid, translating to roughly 50-150 injections per foot, and symptomatic relief lasts as few as 1.5-6 months, making the treatment burden of regular maintenance injections substantial. Thus, improved methods of treatment are needed.

Accordingly, the current disclosure encompasses microneedle arrays, also termed protrusion array devices (PADs), with therapeutically relevant (µg/cm2) dose delivery capability for human skin. Exemplary microneedle arrays can be found in Applicant's copending patent applications U.S. patent application serial nos. 14/788,439 and 14/963,137, which are incorporated herein by reference. For example, using fluoresceinloaded PADs, it can be demonstrated that these PADs can be relatively painless and effectively form intracutaneous depots of polymer and model drug, even in callus areas. In contrast to the discomfort of repeated intradermal injections, PADs can be gently pressed or flicked or otherwise introduced into the skin, delivering hundreds of injections simultaneously at 1.5 mm intervals, for example. A flexible backing conforms to the curvature of the skin, improving both uniformity of penetration and ease of application. A therapeutic toxin dosage form capable of patient-friendly administration of a therapeutic toxin, such as BTX, to multiple skin types can treat a multitude of diseases, disorders, or conditions, including pachyonychia congenita, hyperhidrosis, genodermatosis, epidermolysis bullosa, spastic disorders, facial tics, rosacea, plaque and inverse psoriasis, Frey's syndrome, diabetic neuropathy, headaches, and migraines, many of which currently require multiple standard intradermal injections for botulinum toxin therapy. Additionally, precise administration of BTX into muscle tissue can produce therapeutic focal paralysis in the treatment of a variety of disorders including strabismus, nystagmus, blepharospasm, hemifacial spasm, dystonia, torticolis, cerebral palsy, achalasia, spasmodic dysphonea, stroke-related spasmodic disorders, gastroenteric and urogenital spasmodic disorders, infantile esotropia, and other disorders, as well as in cosmetic procedures to reduce facial wrinkles and expressive disorders. Further, BTX suppression of neural transmission can extend to sudomotor neural firing involved in sweating phenomena. BTX activity can also extend to the inhibition of peripheral nociceptive neurotransmitters, which can provide effective treatment for chronic migraine, chronic post-thoracotomy pain, and other painful disorders with neuropathic component, such as post-herpetic neuralgia, diabetic neuropathy, and trigeminal neuralgia and potentially other conditions involving chronic or neuropathic pain. Further still, the microneedle arrays described herein can provide a highly tolerable delivery platform that can be commercialized both in the form of pre-loaded dosage units and more generally as a drug-naive, loadable device.

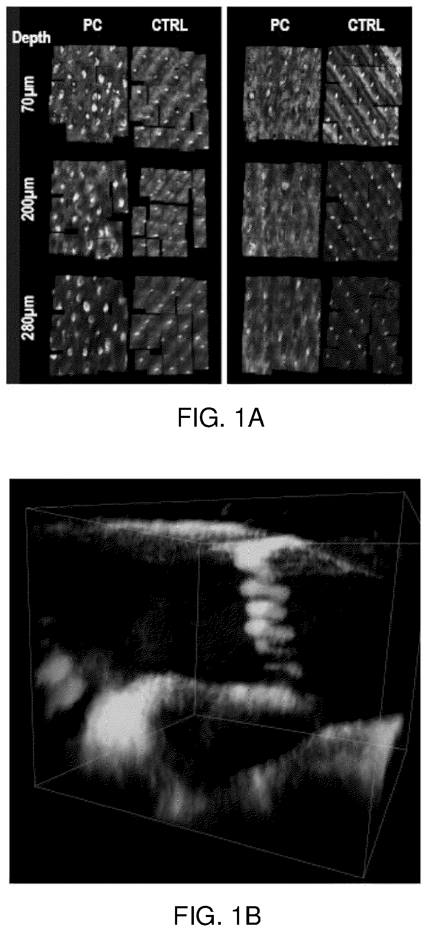

It is noted that with respect to therapeutic toxins or therapeutic neurotoxins, botulinum toxin (BTX) will often be referenced throughout the disclosure for the sake of brevity and clarity as a general example of one such toxin that can be used in the compositions, dosage forms, and methods described herein. This is not intended to be limiting and it will be apparent to one skilled in the art that the disclosure can be extended to other therapeutic toxins, such as those expressly described herein and any other suitable therapeutic toxin. With that in mind, BTX can be effective in treatment of a variety of conditions, such as hyperhidrosis, due, in part, to its ability to inhibit acetylcholine exocytosis in sympathetic sudomotor neurons. However, its role in pain processes is very complex. In some cases BTX can be used as an antinociceptive agent and in reducing neurogenic flares. However, despite its antinociceptive activity, not all pain is responsive to BTX. It is therefore surprising that BTX is not only effective in suppressing hyperhidrotic symptoms in PC, but can further produce remarkable relief of pain in this disease of normally highly recalcitrant and severe plantar pain, despite PC pain showing both neurogenic and nociceptive components. Thus, in some aspects BTX may reduce pain via suppression of sudomotor activity. FIGs. 1A-1B illustrate abnormalities in distribution and physical structure of sweat ducts in PC plantar skin visualized *in vivo* by confocal microscopy, which can be seen as side effects of the disruption in the skin structure associated with this disease. However, pain reduction resulting from BTX therapy may be a result from therapeutically-mediated cessation or reduction in hyperhidrotic sweat production flowing into structurally compromised sweat ducts, which can cause pain or pain-related phenomena. It should also be noted that the PC causative keratins are expressed in sweat glands. Providing an avenue by which BTX therapy might be provided in a brief and tolerable procedure offers the prospect of real and accessible relief to patients suffering from this painful and debilitating disease, with potential extension to other indications with much larger patient populations.

The dynamics of skin penetration by microneedles can be complex and some elements of this process may be readily influenced by the design of needle arrays and application methods. For instance, the general viscoelastic fluid behavior of human skin can dictate that application velocity strongly influences penetration depth, and that the force required to seat a microneedle patch to penetrate the skin can be proportional to the number of needles per unit area. Needle geometry can also be an important factor in penetration efficiency and pain response in normal skin. Thus, the microneedle arrays described herein can have sharp tips and compound curves, which can enable these microneedle arrays to penetrate both normal and hyperkeratotic skin, maximizing both tolerability and delivery.

Further, the microneedle arrays disclosed herein can be preloaded with cargo of choice (e.g., a therapeutic toxin, plasmids, antigens, etc.) and stored dry without loss of activity, stabilized by a polyvinyl alcohol (PVA), or other polymer matrix. The current microneedle arrays are also flexible, inexpensive to manufacture, and can deliver therapeutically relevant doses in a single application. Where the microneedle arrays are biodegradable/bioerodible, they can also be self-blunting and reduce the burden of sharps waste and the related risk of accidents. While microneedle technologies typically have a relatively low cargo capacity compared to hypodermic injection, in the case of BTX, the potency of the drug cargo negates this limitation. For example, PC can be responsive to a 1.3 unit/cm² BTX dose density, which is orders of magnitude below the maximum loading capacity of the current microneedle arrays.

Accordingly, the present invention provides microneedle arrays, associated systems, and methods that can be used to deliver various therapeutic agents. Again, while specific reference will be made herein to botulinum toxin or botulinum neurotoxin, other therapeutic toxins and agents can also be used. In some examples, the therapeutic toxin or agent can be a biologically produced therapeutic toxin or agent. Biologically produced therapeutic agents can generally be those toxins, proteins, nucleic acids, or the like that are produced by or derived from a natural or biological source, such as a plant, bacterium, fungus, or the like. Any such biologically produced therapeutic toxin or agent can also be used and is considered within the scope of the current disclosure.

Therapeutic toxins, peptides, proteins, nucleic acids, or the like can be obtained from a variety of sources, such as bacteria, coral, algae, fungus, snakes, spiders, scorpions, jellyfish, venomous fish, mollusks, amphibians, lizards, the like, or a combination thereof. Non-limiting examples of therapeutic toxins, peptides, proteins, and/or other agents can include cyanotoxins, such as anatoxin-a, lyngbyatoxin-a, aplysiatoxins, and other toxins produced by cyanobacteria; dinotoxins, such as saxitoxins and gonyautoxins, and other toxins produced by dinoflagellates; necrotoxins, causing necrosis or cell death, comprising toxins found in the brown recluse spider, venom of the rattlesnake and other vipers, pore forming toxins of necrotizing fasciitis bacteria; neurotoxins, including toxins that disrupt ion channel conductance, comprising tetrodotoxin, chlorotoxin, conotoxin, botulinum toxin, tetanus toxin, anatoxin, bungarotoxin, carambotoxin, curare poisons, and those found in the venom of the black widow spider, jellyfish, elapid snakes, venomous fish, mollusks, and amphibians, coral and some algae; myotoxins found in snake and lizard venoms; cytotoxins, such as ricin, apitoxin, and mycotoxins, including aflatoxins, ochratoxins, citrinin, ergot toxins, patulin, fumonisins, trichothecenes, zearalenone, beauvercin, enniatins, butenolide, equisetin, fusarins, batroxobins, batrachotoxins, cobrotoxins, crotamines, didemnins, deltorphins, exendins, gephyrotoxin, hannalgesins, histrionicotoxins, opitoxins, phycotoxins, scorpion toxins (such as scorpion β-toxins, etc.), spider toxins (such as co-agatoxins, psalmotoxins, etc.), the like, or a combination thereof. A number of these and other similar therapeutic agents that can be used herein are described in U.S. Patent Numbers 4493796, 4925664, 5514774, 5672682, 5719264, 5739276, 6165500, 7244437, 7608275, 7833979, and 8377951 and US Patent Publication Numbers 2003/0109670, 2004/0192594, and 2007/0191272, each of which is incorporated herein by reference. Further any other material with similar pharmacological utility in treating pain, spasm, hyperhidrosis, or other focal neurologic effects can also be used.

In one specific example, the therapeutic toxin can be botulinum toxin. Precise administration of toxin into muscle tissue can produce therapeutic focal paralysis in the treatment of a variety of disorders including strabismus, nystagmus, blepharospasm, hemifacial spasm, dystonia, torticolis, cerebral palsy, achalasia, spasmodic dysphonea, stroke-related spasmodic disorders, gastroenteric and urogenital spasmodic disorders, infantile esotropia, and other disorders, as well as in cosmetic procedures to reduce facial wrinkles and expressive disorders. Further, BTX suppression of neural transmission extends to sudomotor neural firing involved in sweating phenomena, and intradermal and subcutaneous BTX administration are established therapies for reduction of hyperhidrosis. BTX activity is also understood to extend to the inhibition of peripheral nociceptive neurotransmitters, understood to underlie its use as an effective treatment for chronic migraine, chronic post-thoracotomy pain, and other painful disorders with neuropathic component such as post-herpetic neuralgia, diabetic neuropathy, and trigeminal neuralgia and potentially other conditions involving chronic or neuropathic pain. Botulinum toxin can include botulinum toxin type A, botulinum toxin type B, botulinum toxin C1, botulinum toxin C2, botulinum toxin D, botulinum toxin E, botulinum toxin, F, botulinum toxin G, homologues thereof (i.e. at least 70% homologous, 80% homologous, or 90% homologous), derivatives thereof, or any synaptosomal-associated protein (25 kDa) (SNAP-25) cleaving agent, synaptobrevin II cleaving agent, or syntaxin I cleaving agent, or combinations thereof. SNAP-25, synaptobrevin II, or syntaxin I can each be target substrates for BTX and can be cleaved by BTX. Without being bound to a particular interpretation, it is understood that the botulinum toxins can cleave SNAP-25 within neural synapses, which is required for normal vesicle fusion and release of acetylcholine from axon endings in normal motor neuron activity. The loss of this protein results in a characteristic flaccid paralysis typical of botulinum poisoning. Thus, any SNAP-25, synaptobrevin II, or syntaxin I cleaving agent can be considered within the scope of a botulinum toxin, unless otherwise specified. In one specific embodiment, administration of botulinum toxin A (BTA) is provided. It is noted that when specific reference is made to a single serotype of botulinum toxin, such as BTA, such reference can also include homologues and derivatives of the same, unless otherwise specified. In another specific embodiment, administration of botulinum toxin B (BTB) is provided. In yet another specific example, administration of a combination of BTA and BTB is provided.

In some embodiments, biologically active BTX can be obtained from a variety of sources. For example, BTX can be obtained from a variety of species of the genus *Clostridium.* Such species can include *Clostridium botulinum* (such as groups I, II, III, IV), *Clostridium butyricum, Clostridium baratii,* or a combination thereof, for example. Any one of these sources, or other suitable commercial source of BTX, or combinations thereof, can be used.

BTX concentrations are generally defined as units. One unit (U) of BTX is defined as the LD₅₀ upon intraperitoneal injection into female Swiss Webster mice weighing 18-20 grams each. In other words, one unit of BTX is the amount of BTX that kills 50% of a group of female Swiss Webster mice under the conditions described. A therapeutically effective amount of BTX can depend on the condition it is being used to treat. For example, a therapeutically effective amount of BTX can be an amount that is sufficient to inhibit acetylcholine exocytosis in sympathetic sudomotor neurons. In another example, a therapeutically effective amount can be an amount that is effective at treating and/or reducing the symptom of pain associated with a disease, disorder, or condition, such as a skin disease, disorder, or condition, such as pain associated with the skin. In another example, the therapeutically effective amount can be an amount that is sufficient to decrease blistering resulting from a disease, disorder, or condition, such as a skin disease, disorder, or condition. In yet another example, the therapeutically effective amount can be an amount that is sufficient to reduce sweat production at a treatment site. In an additional example, the therapeutically effective amount can be an amount that is sufficient to reduce skin wrinkles. In a further example, the therapeutically effective amount can be an amount that is sufficient to induce paralysis of subcutaneous muscle adjacent to the site of administration. In some examples, the therapeutically effective amount can be an amount that is sufficient to produce a digit abduction score (DAS), or equivalent, of greater than or equal to 1, greater than or equal to 2, greater than or equal to 3, or greater than or equal to 4 within 24 hours or within 36 hours. The DAS assay is an assay where a toxin is introduced into the gastrocnemius muscle and the amount of paralysis is scored by observation of the ability of the test subject to abduct digits.

Thus, in some examples, a therapeutically effective amount can be an amount from about 0.001 units to about 3,000,000 units. In other examples, the therapeutically effective amount can be from about 0.001 units to about 10,000 units. In other examples, the therapeutically effective amount can be from about 0.01 units to about 300 units. In other examples, the therapeutically effective amount can be from about 0.1 units to about 50 units. In other examples, the therapeutically effective amount can be from about 0.1 units to about 10 units. In other examples, the therapeutically effective amount can be from about 1 unit to about 100,000 units. In other examples, the therapeutically effective amount can be from about 1 unit to about 10,000 units. In other examples, the therapeutically effective amount can be from about 10 units to about 1,000 units. In yet other examples, the therapeutically effective amount can be from about 100 units to about 800 units.

Topical and transdermal delivery, such as via microneedle arrays, can reduce a major barrier to BTX treatment of hyperhidrosis and associated pain and blistering in pachyonychia congenita (PC), as well as other cutaneous disorders. PC skin is understood to be mechanically compromised with fragility due to weakened intermediate filaments resulting from mutant keratin expression. Mechanical stress (e.g., walking) induces the expression of these PC-constitutive keratins, promoting a destructive downward disease cycle.

Infrared micrographs of PC affected tissue can show unusual sweat duct hypertrophy and morphological irregularities and these findings present the intriguing possibility that PC pathophysiology can result from impaired or occluded sweat ducts associated with hyperkeratosis, and that this characteristic might contribute to development of pain and blistering associated with PC, in a manner similar to miliaria profunda. Such a mechanistic connection between sweat production and PC pathogenesis may partly explain the observed striking relief of pain and blistering symptoms following sudomotor suppression by BTX therapy. BTX can similarly provide relief from not only hyperhidrosis, but also pain in related conditions such as genodermatosis and epidermolysis bullosa simplex (EBS), which also involve mutations in keratins (K5 and K14), which form intermediate filaments.

BTX can act to block hyperhidrosis through cleavage of SNARE proteins that mediate synaptic acetylcholine release, thus inhibiting activation of postsynaptic cholinergic receptors that trigger eccrine sweat production. BTX is arguably the most lethal toxin known, with an LD-50 in the ng/kg range, and use of this material for localized intradermal injection to treat hyperhidrosis involves delivery of minute quantities across a large skin area through a multitude of closely spaced intradermal and/or subdermal injections of a dilute solution. Thus, this complex procedure of injections can represent a very substantial barrier to patients suffering an already highly painful skin disorder. Accordingly, topical and/or transdermal administration can provide a much more tolerable delivery of BTX through the use of near-painless delivery via soluble microneedles, which are ideally suited to delivery of minute quantities of highly potent agents to the skin.

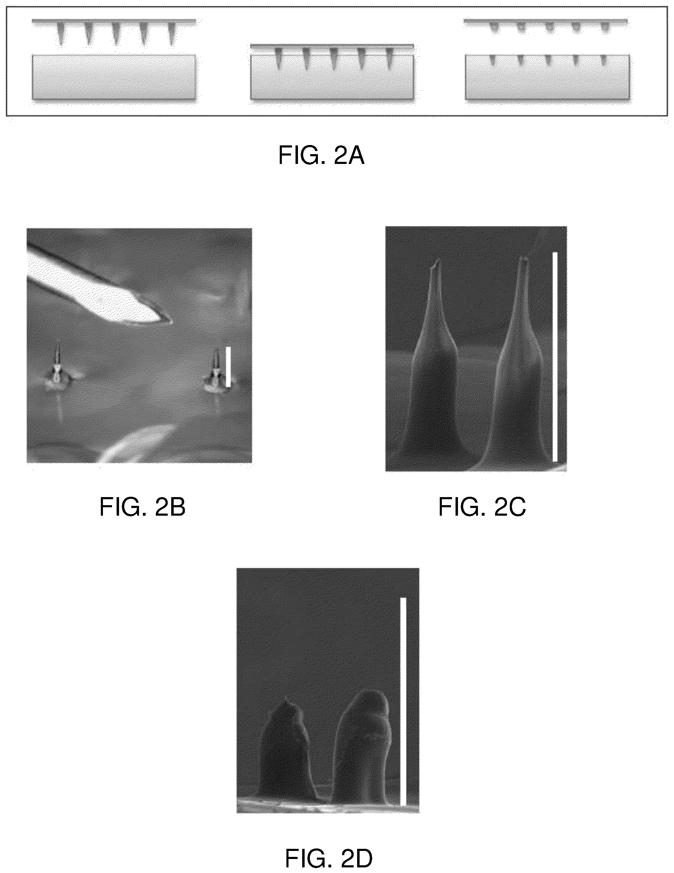

Such microneedle arrays can include a base portion or base layer and a plurality of microneedles attached to, or projecting from the surface of the base portion or base layer. In some examples, the base portion can be a polymer layer. The microneedle array can be part of a transdermal dosage form for treating a disease or disorder in a subject. Thus, a therapeutically effective amount of an active agent, such as BTX, can be loaded to the microneedle array for administration to the subject. The microneedles can be applied to a skin surface of a subject in a manner sufficient to embed the microneedles into the skin surface. In some embodiments, the base portion of the microneedle array can then be separated from the microneedles such that the microneedles remain embedded in the skin surface and the base portion can be removed from the skin surface. In such examples, the microneedles can be maintained in the skin surface until the microneedles are absorbed by the subject. (See FIG. 2A) In other embodiments, the base portion and the microneedles can remain connected. In either case, as can be seen in FIG. 2B, the size of the individual microneedles can be small as compared to the tip of a hypodermic need. FIG. 2C illustrates an enlarged image of some exemplary biodegradable microneedles prior to application to the skin. As illustrated in FIG. 2D, after administration to the skin, the microneedles can biodegrade to release a therapeutic payload. While it is not necessary that the microneedles be biodegradable, having biodegradable microneedles can eliminate a number of safety concerns associated with non-biodegradable microneedles.

In some examples, the microneedle arrays or other delivery vehicle can provide topical and/or transdermal delivery of the therapeutic toxin. It is also noted that either topical or transdermal delivery can be used to provide systemic and/or local dose of a therapeutic toxin. In some examples, topical and/or transdermal delivery can provide a localized (i.e. non-systemic) dose of the therapeutic toxin. In yet other examples, the topical and/or transdermal delivery can provide a systemic dose of the therapeutic toxin.

A major obstacle to transdermal delivery of certain active agents is the challenge of getting the active agents through the *stratum corneum* barrier. The natural function of the *stratum corneum* is to prevent water loss and exclude external agents from entering the body with a molecular cut off of about ~500 Da. The microneedle arrays of the present invention can effectively circumvent this barrier by direct skin penetration. Further, the microneedle arrays of the present invention can be made of bioabsorbable/biodegradable materials and provide the added benefit that the tips may be left in the skin and can act as reservoirs, releasing the active agents over extended periods of time while the microneedles are dissolved and absorbed into the body of the subject. In some examples, the microneedles can be substantially perpendicular to the base portion.

In some examples, the microneedles can include longitudinal channels, such as internal longitudinal channels and/or external longitudinal channels. In some examples, the microneedles can have an internal longitudinal channel formed within the microneedle that extends through the microneedle from the tip of the microneedle to the base portion, and can have an opening at the top (tip end), bottom (base end), or both. In some examples, the microneedles of the present invention can have at least one external longitudinal channel, which is open along at least a portion of its length to the outside of the microneedle. In some embodiments, the microneedles can have several of these external longitudinal channels. Such external longitudinal channels are distinct from enclosed tubes or internal longitudinal channels that can be present in the microneedles and can have openings at the tops (tip end) or bottoms (base end) but not along the length of the microneedles. However, either of the longitudinal channels (internal or external) can be loaded with the active agents delivered by the microneedle arrays. The external longitudinal channels can act to increase the surface area of the microneedle thereby increasing the rate of release of the active agent into the subject. Further, the internal and/or external longitudinal channels can also act to facilitate the loading of the microneedles with the active agent or other compounds.

The microneedles of the microneedle array can have any suitable length. In some examples, the microneedles can have a length from about 1 µm to about 10,000 µm. In yet other examples, the microneedles can have a length from about 50 µm to about 1,000 µm. In still other examples, the microneedles can have a length from about 75 µm to about 500 µm. In some examples, the microneedles can have the lengths described after trimming the microneedles to a desired shape and/or bevel. In some examples, the microneedles can be cut or trimmed to produce a sharpened tip that is suitable to easily penetrate the *stratum corneum.*

The microneedle array can have any suitable number of microneedles, depending on the size and distribution of the microneedles. In some examples, the microneedle array can have from about 1 microneedle to about 25,000,000 microneedles. In some examples, the microneedle array can have from about 10 microneedles to about 200 microneedles. In yet other examples, the microneedle array can have from about 50 microneedles to about 500 microneedles. In yet other examples, the microneedle array can have from about 100 microneedles to about 1000 microneedles. In yet other examples, the microneedle array can have from about 500 microneedles to about 50,000 microneedles. In still additional examples, the microneedle array can have from about 10,000 microneedles to about 10,000,000 microneedles.

The microneedle arrays can have a variety of distributions of microneedles. For example, in some cases, the microneedles can be spaced on the base portion at a density of from about 1 microneedle per square centimeter (cm²) to about 2500 microneedles per cm². In other examples, the microneedles can be spaced on the base portion at a density of from about 10 microneedles per cm² to about 100 microneedles per cm². In other examples, the microneedles can be spaced on the base portion at a density of from about 50 microneedles per cm² to about 200 microneedles per cm². In yet other examples, the microneedles can be spaced on the base portion at a density of from about 100 microneedles per cm² to about 1000 microneedles per cm². In still other examples, the microneedles can be spaced on the base portion at a density of from about 500 microneedles per cm² to about 2500 microneedles per cm².

The microneedle array can be fabricated to simultaneously apply needles to a range of skin surface areas. For example, the microneedle arrays can be fabricated as a continuous sheet, which can optionally be sub-divided into smaller unit doses. In some examples, the microneedle array unit dose can be fabricated to have a surface area or to cover a skin surface area from 1 mm² to 20 cm² or from 10 cm² to 80 cm². In yet other examples, the microneedle array unit dose can be fabricated to have a surface area or to cover a skin surface area from 50 cm² to 150 cm², or from 100 cm² to 1 m². In one specific embodiment, the unit dose can have a surface area or cover a skin surface area from 1 cm² to 350 cm². Unit dose size can be preselected to be appropriate for treating the skin surface of a particular body part, such as the palm of the hand, the sole of the foot, or the front or back torso, for example. Further, the flexible sheets of microneedles can be cut into shapes convenient for application to a selected body part. Thus, the microneedle array can have the shape of a circle, an oval, a triangle, a square, a rectangle, a trapezoid, a rhombus, a crescent, a polygonal shape, or any other suitable shape for a particular application. As one specific example, a crescent shaped unit dose can be fabricated to facilitate application around the eye. Similarly, a mask shape can be produced, with holes cut out to accommodate eyes, nose, and/or mouth. Alternatively, a preselected shape can be dispensed as the base layer and needles subsequently produced from that base layer of a preselected shape.

As another specific example, the microneedle array unit dose can be produced in a preselected shape without any requirement for cutting the base layer. For example, a polymer film can be dispensed in a specific shape to form the base portion or base layer by printing, screen printing, microgravure, jet-printing, use of a peelable template, or any other method of applying a film of liquid to a substrate material to produce a shape. In some examples, needles can be formed by contacting a pin template, or equivalent, to the polymer film and withdrawing the pin template concomitant with evaporative drying of the film to form an array of projections or protrusions by any of the methods described herein. In some further examples, the preselected base layer can be formed by cutting or trimming the protrusions to a desired length and optionally removing the supporting substrate. Notably, there is no requirement that the preselected shape be a continuous uniform base layer, and in some embodiments, gaps in the base layer to produce holes in the resulting shape may be desirable. In some examples, such holes can be positioned so as to facilitate separation into sub-units, as perforations, or to further influence the flexibility or rigidity of the device, the vapor-permeability, the weight, materials cost, and other properties of the unit dose, as will be recognized by one of ordinary skill in the art.

Similarly, the pin template need not make contact with all portions of the polymer film, and in such cases a base layer that includes "blank" zones without needles can be produced. Further, in some examples, the pin template can include pins of different spacing and geometry such that a product can be formed having a non-uniform or gradient density of protrusions, including areas with no protrusions at all. Further there is no requirement that the base layer thickness or composition be uniform, such that unit doses including zones of different polymer composition or coloring or other dissolved or suspended materials can be produced. In some examples, thicker or compositionally varied zones of underlying polymer base layer can impart specific benefits including but not limited to: coloration, visual indicators of active portions, flexibility or rigidity, protrusion shape or dimensions, protrusion rigidity, film adherence to skin, alignment or reference marks, detents or holes to facilitate use in applicator mechanisms, indicators of active or inactive surface, temporary colorant transfers to identify treated skin areas, identification of specific product types and/or a variety of other benefits as will be recognized by one of ordinary skill in the art.

In addition, there is no requirement that the base layer be compositionally continuous or flat. In some examples, the base layer can be discontinuous or can be uniformly or non-uniformly curved or textured. In some examples, curvature of the base layer may require that the pin template be formed with matching curvature, that multiple segments of template be introduced independently, or that the motion of the template be non-linear or complex with respect to the substrate while withdrawing it to form protrusion structures. In some examples, the substrate can further possess indentations or texture such that after application of the polymer solution and subsequent processing to form dried protrusions and base layer, the base layer retains the pattern of the substrate in the manner of a cast molding. Such molded features may or may not underlie the area of the device bearing protrusions, and may further include text or other identifying marks such as alignment or registration marks, voids, indentations, or protruding zones that facilitate attachment or alignment of the device to an applicator, or integrally molded labels, warnings, or other visual indicators of active or inactive zones of the device or zones of differing composition or function, for example, including "this side toward skin" or other instructional indicators. Further, in some examples, texturing of the base layer resulting from micromolding by the underlying substrate can influence protrusion shape or structure and can further influence the rigidity, flexibility, stretchability, elasticity, or facilitate curvature of the resulting device. In some additional examples, the base layer can further be cast to include or conjoin to previously or separately formed components, for example radio-frequency identification RFID, or electronic sensor devices, or identifying tags or visual indicators, including bar-codes and other scanned identifiers, among others.

The microneedles can also be configured to have a lower portion and an upper portion, the lower portion being adjacent to the base portion and the upper portion being opposite the base portion, or at the tip of the microneedle. In one embodiment, the lower portion and the upper portion of the microneedle can be compositionally distinct. In one embodiment, the active agent can be present only in the upper portion of the microneedle. In another embodiment, the active agent can be present only in the lower portion of the microneedle. In another example, the upper portion and the lower portion can have different concentrations of the active agent. In yet a further embodiment, the microneedle can include two different active agents. In some examples, where the microneedle includes two different active agents, a first active agent can be present in the lower portion and a second active agent can be present in the upper portion. In yet other examples, the active agent can have a gradient concentration from the tip of the microneedle to the base of the microneedle. In some examples, the gradient concentration can be from a higher concentration at the tip to a lower concentration at the base. In other examples, the gradient concentration can be from a lower concentration at the tip to a higher concentration at the base. It is noted that the gradient concentration need not be a linear gradient, but can be a step gradient (i.e. having 2, 3, 4, or more steps), or other suitable gradient.

As previously discussed, two or more different active agents can be included in the microneedle array. A number of suitable active agents can be administered with BTX. For example, the BTX can be administered with an analgesic, such as paracetamol, NSAIDs, suitable opioids, or a combination thereof. The BTX can also be administered with other suitable agents, such as collagen, hyaluronic acid, the like, or combinations thereof.

In some examples, the microneedles of the microneedle arrays can be made of bioabsorbable/biodegradable materials and, in some further examples, can also include materials that can hydrate to form an intradermal and/or subcutaneous depot upon administration. Non-limiting examples of bioabsorbable/biodegradable materials that can be used include polyvinyl alcohol, polyvinylpyrrolidone, carbomers, polyacrylic acid, polyoxyethylene/polyoxypropylene copolymers, other copolymers, albumins, casein, zein, collagen, other proteins, glucose, sucrose, maltose, trehalose, amylose, dextrose, fructose, mannose, galactose, other sugars, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, other sugar alcohols, chondroitin and/or other glycosaminoglycans, inulin, starches, acacia gum, agar, carboxymethyl cellulose, methyl cellulose, ethyl cellulose, alginates, carrageenan, cassia gums, cellulose gums, chitin, chitosan, curdlan, gelatin, dextran, fibrin, fulcelleran, gellan gum, ghatti gum, guar gum, tragacanth, karaya gum, locust bean gum, pectin, starch, tara gum, xanthan gum, and other polysaccharides, and functionalized derivatives of any of the above, copolymers thereof, or mixtures thereof. The bioabsorbable/biodegradable materials are generally only limited by the ability to create a viscous solution in a solvent that can volatilize during formation of the fiber-like needle structure, and/or the property of drying to form a glassy or non-crystalline solid. In the present invention, polymeric or glassy materials that are water-soluble can have some advantages due to their ability to hydrate in skin and be biologically cleared through absorption. Further, glassy polymers can stabilize biomolecules in dried form, providing a useful benefit of increased storage stability of the active agent or drug cargo incorporated into the microneedles. In one embodiment, the bioabsorbable/biodegradable material can be polyvinyl alcohol. When the bioabsorbable/biodegradable polymer is polyvinyl alcohol, the weight average molecular weight (Mw) of the polyvinyl alcohol can be over 10,000 Mw.

The base portion of the microneedle arrays of the present invention can be made of any material known in the art onto which the microneedles can be attached, or from which the microneedles can extend, and optionally subsequently separated. In one embodiment, the base portion of the microneedle array can be made of the same bioabsorbable/biodegradable material as the microneedles. Typically, the base portion can be flexible to facilitate application to curved skin surfaces.

The microneedles of the microneedle arrays can also include an indicator capable of providing visual verification of microneedle placement in the skin surface. The indicator can be incorporated into the body of the microneedle, loaded onto the exterior of the microneedle, or loaded into the microneedle along with the drug, or any combination thereof. The indicator can, but need not, be present in all of the microneedles of the microneedle array. Further, the indicator can be present in the microneedles of the microneedle array so as to form an image when the microneedles are embedded in the skin. In one embodiment, the image of the microneedle array can be specific to the particular active agent delivered by the microneedle array, thereby allowing a practitioner to know not only if the microneedles had been properly embedded into the skin of the subject, but also what type of active agent was received by the subject. This can be particularly advantageous when the subject may be in need of receiving multiple active agents. Furthermore, the image can be that of a creative or fun nature for the enjoyment and purposes of children or others receiving vaccination as such visual indicators will remain as a "tattoo" on the skin until the microneedles dissolve or are absorbed.

Generally, any biologically acceptable indicator known in the art can be used. In one embodiment, the indicator can be a dye. In one aspect, the dye can be deposited onto the exterior of at least one of the microneedles on the microneedle array using an ink-jet apparatus, by contacting the needles with a surface upon which the dye is previously deposited in a pattern, or by contacting dye with needle structures individually in an indexed fashion. In another embodiment, the indicator can be one that is only visible upon direct application of a light source. In one aspect, the indicator can be visible only after application of ultra-violet and/or infrared light. The indicator can also be a biological indicator. In one embodiment, the indicator can be a fluorophore such as fluoresceine. In another embodiment, the indicator can be a bioluminescent enzyme such as a luciferase.

Generally, the needles can be loaded with an active agent such as those described above. This can be done in a number of ways. For example, in some cases, the microneedle can be loaded with active agent by including the agent in the bulk material from which the needle structures are formed. In some examples, this can be done in a plurality of layers, some or all of which can be distinct in concentration and/or composition. In other examples, the microneedle can be loaded with active agent by adding material to the surface of a polymer film from which the needles are pulled. In yet other examples, the microneedle can be loaded with active agent by loading the needles after they are fully formed. Of course, a combination of these methods can also be used.

In the case in which the bulk material is loaded, an active agent payload, such as a fluorescein model drug, can be co-dissolved with the polymer or dry component(s) of the film, or it may be dissolved (or suspended if poorly soluble) in this viscous solution prior to dispensing as a film from which needle structures will be pulled or molded. An active agent included in this manner will be distributed throughout the needle structures and the backing material in rough proportion to the quantity of material in each part.

In the case in which payload material is added to the surface of the polymer solution film prior to forming the needle structures, without wishing to be bound to a particular interpretation, it is observed that material on the surface of the film from which needle structures are formed is preferentially drawn into the needle structures pulled from this film, and needles thus formed are subsequently found to be enriched in any material that was thus surface-loaded relative to the quantity of that material in an equivalent volume of the backing material. Thus, surface-loading of the film provides an enhancement of the efficiency of use of an active agent. For example, an ethanolic solution of 1% wt fluorescein model drug can be applied to the surface of a 100µm thick spread film of 20% PVA solution at a loading of about 20 uL per square cm, prior to needle formation. The resulting needles pulled from this surface-loaded PVA film are strongly colored by the fluorescein payload, while the backing material remains relatively pale in coloration, indicating that the majority of the fluorescein is incorporated into the needles. Similarly, a second, thinner PVA film containing a drug can be applied in the same manner, or a film containing material to be incorporated besides PVA, with similar preferential incorporation into the needle structures.

In the case in which payload material is loaded into/onto the needle structures after these structures are fully formed, the needles are brought into brief contact with a solution containing active agent dissolved or suspended in a volatile phase, and subsequently this phase is evaporated, depositing the active payload as a component of the needle structure. In the case that the volatile phase will also swell or dissolve the material of which the needle is formed, for example a solution containing water in contact with a PVA needle, it is understood that the needle can additionally imbibe the solvent material, and can carry payload material into the matrix of the structural material that forms the needle. For example, a 1% fluorescein solution in 50% ethanol/water solvent will efficiently wet the surface of a dry PVA needle structure, being drawn into superficial channels along the needle length. Because PVA is hydrated and solubilized by water, a needle thus exposed becomes flexible and pliant, indicating that the water has diffused into the bulk PVA material comprising the needle structure. Coloration of a needle thus treated indicates that the fluorescein payload material also penetrates into the needle structure. Additionally, though such loading contact time is brief, typically less than one second, it is understood that some dissolution and re-deposition of the PVA needle material must take place, which may further incorporate payload into the bulk of the needle structure. Generally, the bulk loading solution will not directly solvate the PVA microneedle structures, but will contain a volatile component such as ethanol, such that the needle structures may be wetted by momentary contact with the solution, and then after removal the volatile component can evaporate rapidly relative to the water component, leaving needles wetted with a small quantity of water which can then be absorbed and evaporated without producing any substantial dissolution of the structures, but rather briefly hydrating them, during which action the payload is incorporated.

In some examples, when a payload material is loaded onto the preformed needle structures, the payload solution can also include additional inactive, excipient, or adjuvant materials including but not limited to any of the materials recited above from which needles might suitably be manufactured. Such additional components of the drug loading solution can have the potential to influence dissolution and drug diffusion characteristics during and after deposition into the skin. Further, such over-coated materials can influence stability of the loaded active agents, the microneedle structures, or the entire product, or can contain additional colorants, markers, or indicators. For example, an additional indicator can be used that indicates the presence of moisture that might compromise the rigidity of the microneedles or the stability of the loaded drug, or a material that provided a color change indicating that the product had been stored outside of a suitable temperature range, or a material that provided a visual indicator that the microneedles had been used, or that the needles had effectively delivered the payload into skin. Further, in some examples, such indicator materials can be loaded onto an area of the base layer that does not contain needle structures such that the indicator material need not be delivered as part of the needle payload. Loading solutions can also include components to alter viscosity, surface tension, solvation, dissolved excipient load and/or other characteristics to facilitate wetting or non-dissolution of the needle structures or deposition of an active payload in the needles and/or in an excipient layer.

In another embodiment, a method of treating a disease, disorder, or condition in a subject using a therapeutic toxin, such as botulinum toxin, is described. The method can include topically or transdermally administering a therapeutically effective amount of botulinum toxin to a subject. In some examples, the botulinum toxin can be administered using a microneedle array, as described herein. In other examples, the therapeutic toxin can be administered using a gel, cream, ointment, patch, or the like. In other examples, the method can include administering the toxin in a gel, cream, or other preparation and separately administering the microneedle array before and/or after administering the gel, cream, or other toxin preparation.

The disease, disorder, or condition can include PC, hyperhidrosis, upper motor neuron syndrome, blepharospasm, strabismus, chronic migraine, bruxism, cancer, any other disease, disorder, or condition described herein, including the symptoms thereof, such as pain, itch, the like, or a combination thereof.

The subject can be a human, a mammal, or any veterinary subject. The therapeutically effective amount of therapeutic agent can depend on the disease, disorder, or condition being treated, the mode of administration, the therapeutic agent being administered, and the like. In some examples, the therapeutically effective amount can be administered via an effective dosage regimen. The effective dosage regimen can include a single dose or a plurality of doses over a single day or a plurality of days. For example, in some cases, the BTX can be administered from 1 to 100 times per day, from 1 to 50 times per day, from 1 to 24 times per day, from 1 to 12 times per day, or from 1 to 6 times per day.

Where a microneedle array is used to administer the active agent, the microneedle array can be left in place for a variety of times. For example, in some cases, the microneedle array can be left in place for a period of from about 5 seconds to about 24 hours. In yet other examples, the microneedle array can be left in place for a duration of from about 10 seconds to about 12 hours, or from about 30 seconds to about 6 hours. In some examples, the microneedles can be detached from the base portion of the microneedle array to provide sustained delivery beyond the period of time the base portion is left in place.

The frequency of administration and duration of administration can also determine the dosage amount per administration event. For example, in some cases, it can be desirable to administer low doses of the active agent more frequently. In yet other examples, it can be desirable to administered higher doses of the active agent less frequently. Thus, each administration event can be repeated a plurality of times per day, once per day, once or twice per week, once or twice per month, once or twice per three months, once or twice per four months, or the like, while symptoms remain. The dosage amount and frequency can be coordinated to provide a therapeutically effective amount that is not likely to result in a toxic accumulation of the active agent over time.

In some examples, the therapeutically effective amount can provide a reduction in sweat production at a site of treatment, as compared to sweat production prior to or without treatment. In other examples, the therapeutically effective amount can provide a reduction in the symptom of pain, as compared to a level of pain experienced prior to or without treatment. In other examples, the therapeutically effective amount can reduce the number of wrinkles at the site of treatment, as compared to the number of wrinkles prior to or without treatment.

As previously discussed, a therapeutically effective amount of BTX can provide a reduction in sweat production. In accordance with this, the present disclosure also provides a method of monitoring a reduction in sweat production using a moisture-sensitive film, which can result from treating with a therapeutically effective amount of a therapeutic toxin. Further, a moisture-sensitive film for use with the method is also described.

The moisture-sensitive film can include a starch-containing substrate and a thin layer of iodine applied to the starch-containing substrate. Generally, the starch-containing substrate can include paper, fabric, polymeric materials, the like, or combinations thereof. In some specific examples, the starch-containing substrate can be a cellulose or paper based material. In some further examples, the starch can be combined with the raw materials used to prepare the cellulose or paper based material to provide the paper based material with increased dry strength and/or a modified surface texture. In additional examples, the starch can be included as a coating applied to the paper based material. Where a coating is applied to the paper based material, the coating can be any suitable coating that can have any suitable combination of ingredients. For example, the coating can include any suitable combination of pigments, binders, polymeric materials, fillers, the like, or a combination thereof.

In additional examples, the starch-containing substrate can be a polymeric material, such as for example, polyvinyl alcohol, polyvinylpyrrolidone, carbomers, polyacrylic acid, polyoxyethylene/polyoxypropylene copolymers, other copolymers, albumins, casein, zein, collagen, other proteins, glucose, sucrose, maltose, trehalose, amylose, dextrose, fructose, mannose, galactose, other sugars, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, other sugar alcohols, chondroitin and/or other glycosaminoglycans, inulin, starches, acacia gum, agar, carboxymethyl cellulose, methyl cellulose, ethyl cellulose, alginates, carrageenan, cassia gums, cellulose gums, chitin, chitosan, curdlan, gelatin, dextran, fibrin, fulcelleran, gellan gum, ghatti gum, guar gum, tragacanth, karaya gum, locust bean gum, pectin, starch, tara gum, xanthan gum, and other polysaccharides, and functionalized derivatives of any of the above, copolymers thereof, or mixtures thereof. The composition of the starch-containing substrate is generally only limited by the ability to create a viscous solution containing dissolved starch that can be dried to form a glassy or non-crystalline film.

A variety of different starches can be used to make or coat the starch-containing substrate. In some examples, the starch can be oxidized starch, cationized starch, esterified starch, enzymatically denatured starch, the like, or a combination thereof. Generally, any starch or starch derivative can be used that can form a colored complex with iodine in the presence of water.

As one specific, non-limiting example, a starch-containing substrate can be formed by preparing an aqueous solution containing 15% polyvinyl alcohol (Spectrum Chemicals, New Brunswick, NJ) and 1% maltodextrin (Maltrin M040, Grain Processing Corporation, Muscatine, IA), subsequently casting or otherwise spreading the solution into a 200 µm thick film, and evaporating to dryness. The resulting film can be coated with iodine, as described below, to form a moisture-sensitive film or moisture responsive imaging sensor.

In further detail, a thin layer of iodine (I₂) can be applied to the starch-containing substrate. The iodine layer can be applied to the starch-containing substrate in a variety of ways. In some examples, the layer can be coated with iodine without triggering strong color formation by coating with a non-aqueous iodine solution (such as a solution of iodine containing less than 1 wt% water) and subsequent evaporation. In one specific example, a solution of 1% iodine in reagent ethanol (Spectrum Chemicals, New Brunswick, NJ) can be sprayed onto the maltodextrin/PVA film described above and evaporated to dryness. The resulting iodine coated starch/PVA film can produce a strongly colored complex in the presence of sub-microliter quantities of water. If water exposure is spatially discontinuous, such as when the film is pressed against skin and individual sweat ducts are distributed over different areas of the skin surface, a 2-dimensional image will result from the localized hydration of the film in the region of those sweat ducts.

In other examples iodine can be deposited on the film without producing the strongly colored complex by direct condensation of iodine vapor in the absence of liquid water. For example, thermal cash register paper (Thermal Paper Direct Inc, Mahwah, NJ), or similar paper based materials, can have useful quantities of starch for preparation of the moisture-sensitive film. As one specific example, the moisture-sensitive film can be prepared by placing a sample of thermal cash register paper in a glass container with crystalline iodine at 24°C. Over the course of several hours the paper can develop a light brown tint due to iodine vapor condensation onto the paper surface. The resulting moisture-sensitive film can develop a strong purple color with application of moisture, and can readily produce a fingerprint image including darkened spots at the site of active sweat pores. Depending upon the relative moisture of the fingers of the tested individual this image may appear in as little as 5 seconds or may take as long as 1 minute to appear clearly. In other aspects, a longer or shorter contact time may be more optimal to detect a particular moisture level or tissue feature and no specific time requirement is implied herein.

In other yet other examples, the iodine layer can be applied by bringing a second iodine-coated or iodine-rich layer into direct physical contact with the starch-containing substrate. For example, a thin layer of sodium alginate (Manucol DH, FMC Biopolymers, Philadelphia, PA) can be produced by drying a 2% solution of the alginate further containing 0.05% glycerin (Spectrum Chemicals, New Brunswick, NJ) as a plasticizer, and 1% iodine (Spectrum Chemicals) on a polymethylmethacrylate (PMMA) sheet substrate. Alginate alone does not produce a colored complex with iodine. The dried alginate film containing iodine can be peeled from the PMMA substrate and applied to one of the starch-containing substrates described above. The resulting 2-layer moisture-sensitive film can form a strongly colored starch iodine complex locally when a drop of water is applied.

The thin iodine layer can provide an even, brown color on the surface of the starch-containing substrate. This color can be retained for several weeks when stored in a closed, air-tight container. Such containers can include glass, polyethylene, wax, aluminum foil, the like, or combinations thereof, although some metals and other materials, notably iron-containing materials, are sensitive to iodine and rapidly corrode, and are therefore not ideal for storage of the iodine-rich films unless provided with a protective over-coating impermeable to iodine vapor. When the thin iodine layer is exposed to moisture, such as from a fingerprint, foot print, or other skin surface, and associated sweat ducts, the moistened area darkens substantially. Further, in some examples, this darkening can be substantially irreversible, such that when the imprinted and darkened area is left exposed to air, it remains darkened. In contrast, in some examples, the undarkened areas can lighten over time when exposed to air until the brown color is substantially or completely dissipated. Without wishing to be bound by theory, it is believed that this can result from iodine in the presence of water becoming entrapped in the helices of amylose in the starch-containing substrate. This entrapped form of the iodine can be less labile and less subject to loss via evaporation. The evaporation of the non-imprinted areas also serves to increase the contrast of the imprinted areas, making imaging of the imprinted moisture-sensitive film clearer.

It is noted that by applying the iodine layer to the starch-containing substrate, there is no need to apply iodine to the skin surface prior to contacting the skin surface with the moisture-sensitive film. This can provide a more uniform measurement across the moisture-sensitive film as compared to applying iodine to the skin surface and then contacting the skin surface to the starch-containing substrate. However, the starch-containing substrates can be used without an incorporated iodine layer. In these examples, the skin or other skin surface can be pre-coated with iodine and then brought into contact with the starch-containing substrate to produce images similar to those produced by the moisture-sensitive film.

In some additional examples, the moisture-sensitive film can include a backing layer applied to a back side of the starch-containing substrate, the back side being opposite the side of the starch-containing substrate to which the iodine layer is applied. In some examples, the backing layer can provide increased sensitivity in detecting moisture content on a skin surface. In some examples, this can result from greater confinement of the moisture within the moisture-sensitive film rather than allowing the moisture to pass through the moisture-sensitive film. Thus, the backing layer can provide a selectable degree of moisture permeability. For example, in some cases, the backing layer can be a water-impermeable layer. In yet other examples, the backing layer can be a semi-impermeable layer with respect to water. In yet other examples, the backing layer can be a water absorbent material. These different types of backing layers can accommodate different levels of skin moisture. For example, a highly absorbent backing layer can be more suitable for a high moisture sample without overexposing or saturating the signal of the moisture-sensitive film. Conversely, a highly impermeable backing layer can provide increased sensitivity for low moisture samples by preventing the moisture from passing through the moisture-sensitive film.

As previously mentioned, the moisture-sensitive film can be used in a method of monitoring a reduction in sweating or a method of monitoring moisture content on a skin surface. The method can include providing a moisture-sensitive film to a subject and applying a skin surface of the subject to the moisture-sensitive film. In some examples, the skin surface that is applied to the moisture-sensitive film can be a skin surface that has been treated with a microneedle array, such as those described above. In some additional examples, the skin surface can be any skin surface where it is desirable to determine sweat production or skin surface moisture levels for any reason. In some specific examples, the skin surface can be the skin surface of a foot, such as the bottom of a foot.

As described above, the moisture-sensitive film can become darkened upon exposure to skin moisture. Thus, when the moisture-sensitive film is contacted with a sample skin surface, the moisture-sensitive film will darken in proportion to the moisture content of different areas of the skin surface. This can provide a "fingerprint" or "perspirograph" of the sample skin surface that can be imaged and used for monitoring of sweat production over time, which can help verify that a therapeutically effective amount of the therapeutic toxin or other therapeutic agent is being administered to the treatment site. As such, this method can be performed at any suitable interval to monitor the progress of treatments.

A few non-limiting examples are provided below to help illustrate a few of the benefits of the methods, devices, and systems disclosed herein. These examples are provided for illustrative purposes only and are not intended to be limiting.

### EXAMPLES

### Example 1 - Microneedle Array Device Preparation

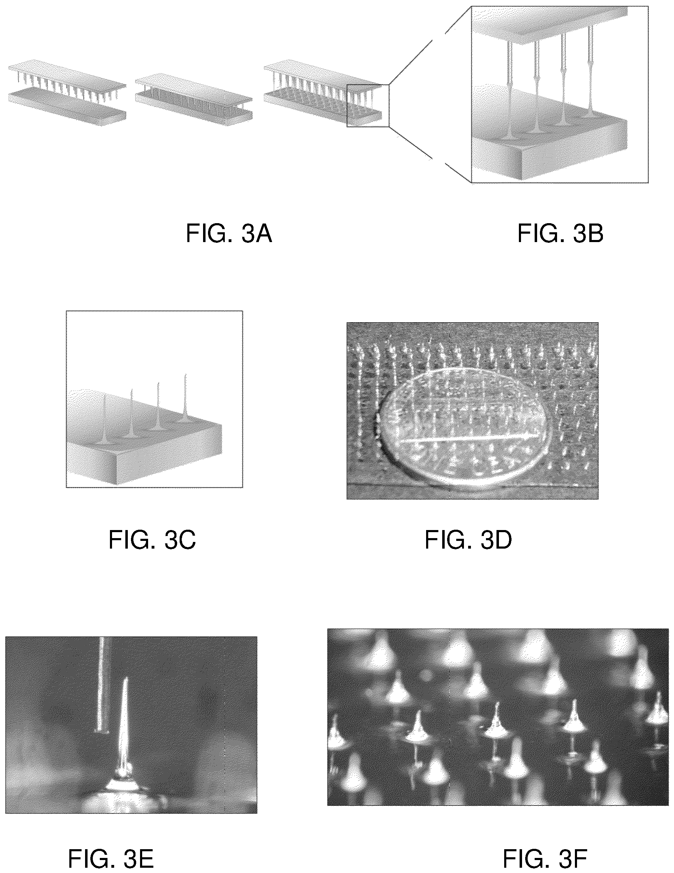

Microneedle arrays were produced by bringing a pin template into contact with a 1 mm thick film of 20% wt. polyvinyl alcohol (PVA) solution (FIG. 3A, left and middle panels), and withdrawing the template under a controlled air flow to produce fiber-like structures with loadable channels (FIG. 3A, right panel and 3B). The dried needle structures are separated from the template, and mechanically trimmed to a uniform height with sharp beveled tips (FIG. 3C). Typical fabrication and drying of microneedle arrays occurs at or below 50°C (as low as 30°C, data not shown). The device is removed from the substrate as a regular array of dissolvable microneedles with an integral material backing (FIG. 3D). Typically, the microneedles on the microneedle arrays can be less than 100 µm thick, with a sharp tip only microns across (FIG. 3E). Microneedle arrays can be loaded with aqueous solutions or suspensions of payload materials, such as nucleic acids, drugs, vaccines, or other therapeutic agents. Multiple payloads can be delivered from separate needle populations on the same array (FIG. 3F). To show the flexibility of loading, phycobiliprotein R-phycoerythrin and fluorescein were alternatively loaded on arrays, demonstrating that multiple cargos can be delivered with a single device (FIG. 3F).

### Example 2 - Sweat Production Fingerprinting

The current inventors have developed a membrane-based adaptation of the iodinestarch complexation commonly used to visualize sweat pores, comprising a thin, flexible film of polyvinyl alcohol (PVA) and maltodextrin. In the presence of iodine, this film rapidly develops color when moistened. This film approach presents substantial advantages compared to the more common approach of using dry starch granules or granules dispersed in oil. The film shows coloration in high resolution, reflecting the homogeneous distribution of the starch in the dry membrane, and the coloration is developed within the film itself such that it can be removed from the underlying tissue substrate and subsequently visualized and permanently recorded (scanned) using standardized electronic methods, avoiding a great deal of visual analysis of spots seen on the irregular surface of the subjects' skin, and promoting quantitative comparisons of sweat levels.

In further detail, membranes prepared by drying polyvinyl alcohol/maltodextrin solutions are responsive to sweating in the presence of iodine. High-resolution images of sweat production with resolvable sweat ducts are produced by this "fingerprinting" method wherein an iodine coated skin surface is pressed against a dried starch/PVA film for several minutes. Areas as small as mouse paws and as large as human feet can be consistently assayed using this method and the resulting "films" can be stored in a dry environment or electronically imaged for more quantitative analysis.

The inventors have further adapted the membrane sensors described above to a paper-based device that does not require the use of PVA or any other separately applied polymer. Briefly, paper, fabric, or any other hydrophilic surface is loaded with a material known to form a colored complex in the presence of iodine and water, typically starch, maltodextrin or similar, although PVA may also be used in this role. The surface is dried, if necessary, and then elemental iodine is deposited onto this surface in an anhydrous fashion that precludes premature complexation. The resulting surface loaded with both iodine and a corresponding agent capable of forming a colored complex, is sensitive to water, rapidly developing a visible coloration upon exposure to minute quantities of water on the order of 1 nL / mm² or less, or tens of pL of water on local exposure to an individual sweat pore. Iodine can be conveniently deposited in an anhydrous manner by direct vapor exposure from the crystalline solid, or by wetting of the starchy substrate by a solution of elemental material in an anhydrous (or nearly anhydrous) solvent such as reagent methanol or ethanol, or similar, followed by evaporative drying of the solvent, which proceeds more quickly than evaporative loss of iodine. The resulting films may be stored in a sealable container or package so as to preclude gradual loss of iodine content and must be stored in sufficiently low-humidity conditions to preclude condensation of liquid water and development of color in the film sensor material. Most commercial paper designed to accept printing includes so-called sizing-agents, imparting a number of benefits, which include uniform and ready absorbence of ink materials. These sizing agents frequently include materials that, like starch, will form colored complexes with iodine in the presence of water, and thereby provide a ready platform adaptable to the moisture sensing devices described herein.

The moisture-sensing films produced as described above are readily calibrated as to detection of specific moisture levels by application of minute quantities of water. A convenient method to deliver minute quantities of water is to introduce the water in the form of a solution in a non-aqueous solvent such as reagent ethanol or methanol. The films described above show no visible coloration upon exposure to a 1µL quantity of neat reagent ethanol. However, a 1 µL aliquot of a 2% solution of water in reagent ethanol is in some embodiments sufficient to produce a readily discernible color change in the paper, uniformly visible over a spot 6 mm in diameter, corresponding to an area of about 28 mm², appearing over about 10 seconds as the ethanol evaporates, leaving the water to soak into the film. The spot thus visualized results from the 20 nL water content of the original 1 µL of solution. Thus color is apparent at a water dose of 0.7 nL / mm². The spot size observed as a colored region resulting from contact with an active human sweat pore can be as small as 0.12 mm in diameter, with an area of 0.011 mm², some 60-fold smaller than the 1 µL calibration spot size, corresponding to a water dose measured from the pore that is in the range of 10 pL. To facilitate quantitation of signal intensity in response to water exposure, it may be desirable to expose each sample or batch of the inventive films described to precise volumes of water solutions of calibrated concentration, such that signal-response data is generated in-situ. Inclusion of one or more such calibration marks ensures that the sensors are functional and may facilitate grading or quality control as well as providing a convenient read-out reference for subsequent use.

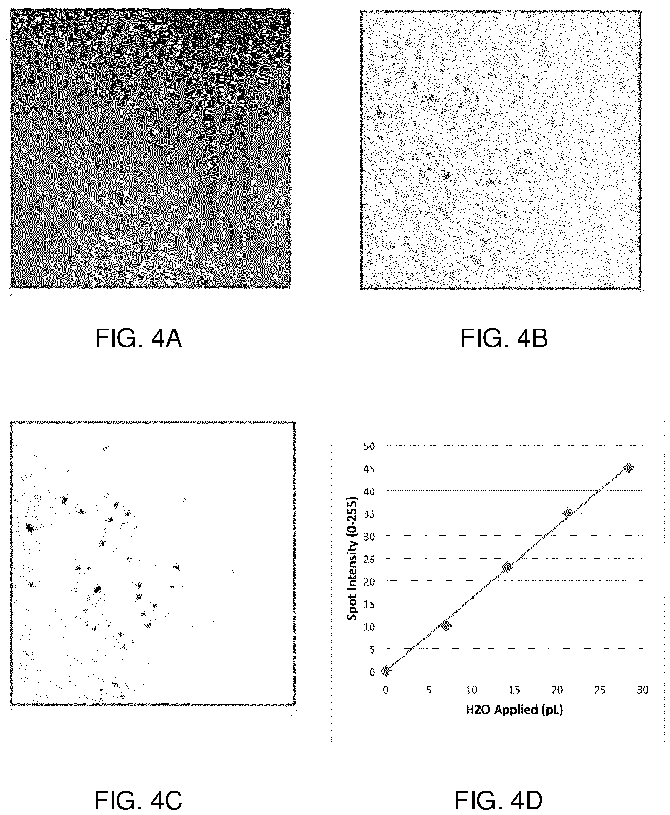

For example, FIG. 4A depicts a PVA film in contact with iodine-treated human palmar skin showing dark spots over sweat pores. FIG. 4B shows the PVA film after removal from the skin. As illustrated in FIG. 4B, some light generalized staining is visible, showing distinct punctate dark spots corresponding to sweat pores. Further, FIG. 4C illustrates an example of threshold detection of active glands for quantitative measurement. FIG. 4D depicts a graph showing water intensity response of a paper-based starch-iodine sensor film scaled to pore size, indicating that approximately 8-10 pL of water produces a measurable response. Water response intensity was quantified by electronically imaging the film using an Epson Reflection V550 scanner run by Epson Scan software version 3.9.2.3US at 24-bit RGB color and 1200 dpi resolution settings. Image signal intensity levels were extracted using open-source ImageJ software version 2.0.0-rc-42/1.50d.

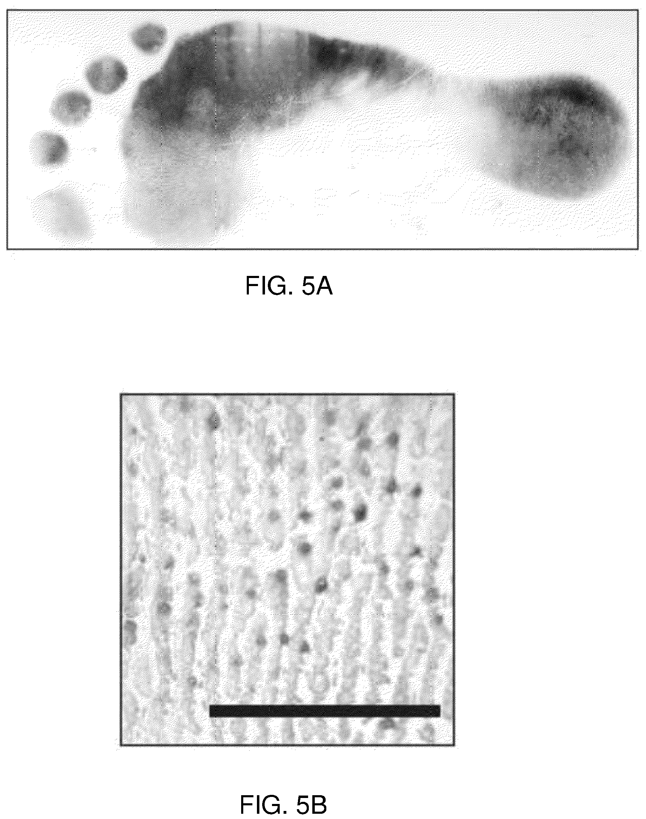

FIG. 5A illustrates one specific example of a footprint demonstrating the capability of this approach to record sweat production over large areas (e.g., entire sole) in a single operation. A close-up view of detail recorded in the film is illustrated in FIG. 5B, with individual pores readily discernable within skin ridges of the plantar surface. Not all pores are active in this healthy volunteer, and light areas correspond to lack of contact with the PVA film and dry sweat duct openings. Ridges are visible due to moisture content, although these are readily discerned versus the more intense coloration of the pores.

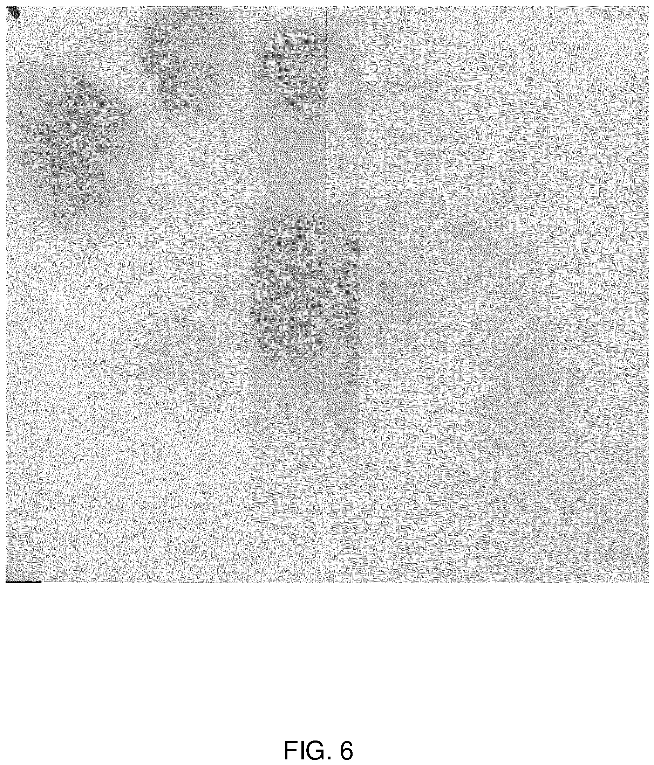

FIG. 6 illustrates two starch-containing paper-based films coupled together with an adhesive film. As can be seen in this image, the adhesive film can provide increased sensitivity in detecting moisture content on a skin surface.

### Example 3 - Sweat Production is Suppressed in Mouse Footpads Administered BTA by ID Injections or Microneedle Array

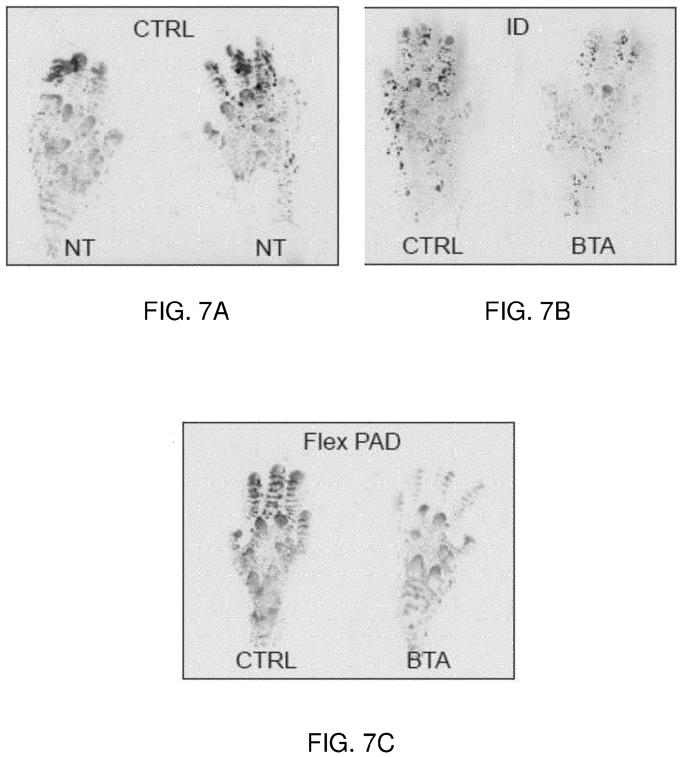

As illustrated in FIGs. 7A-7C, suppression of sweat production following treatment with BTA is evident within 3 minutes of exposure against starch/PVA films. More specifically, negative control footpads without treatment are illustrated in FIG. 7A showing equivalent staining intensity on each foot. FIG. 7B depicts footpads visualized 24 h following intradermal injection of saline negative control (left) and 50 units BTA in saline (right). The BTA-treated paw exhibits lighter staining, consistent with reduction of sweat production. Similarly, FIG. 7C depicts footpads analyzed 4 days following treatment with a blank microneedle array (Flex-PAD) (left foot) application and BTA-loaded Flex-PAD (delivering approximately 50 units BTA, right foot). The paw (right) receiving BTA delivered by the Flex-PAD, shows lighter staining consistent with BTA-mediated reduction of sweat production.

While botulinum toxin is generally not stable in solution for long-term storage, these preliminary results show that sufficient potency is retained for activity. Further, dramatic stability enhancement of biological materials can be accomplished after loading onto microneedles, even at elevated temperatures. In some cases, this can be due to the glassy polymer structure of the PVA substrate material. For additional stability, an alternate loading process can be used such that processing temperatures are held near 5°C to minimize the potential for degradation.

### Example 4 -Botulinum Toxin Delivery Measured Via Digit Abduction Score (DAS) Assay

Female Swiss-Webster (SW) mice were evaluated to observe normal digit abduction reflexes and full normal spreading of digits during tail suspension or "scruffing." The subjects were anesthetized and a single Flex-PAD unit dose, comprising 9 microneedles loaded with a total of approximately 60 ng BTA, was applied to the shaved skin overlying the right gastrocnemius muscle of each subject. Control unit doses loaded with approximately 5 ng USP riboflavin phosphate in place of BTA (control) were identically applied to shaved skin overlying the left gastrocnemius muscle. Unit doses were applied by finger pinch insertion and left in place for 5 minutes. Subsequent inspection of unit doses after treatment showed evidence of substantial erosion of at least 50% of the needles, indicating that while the doses did not likely deliver their full cargo, substantial deposition was achieved.

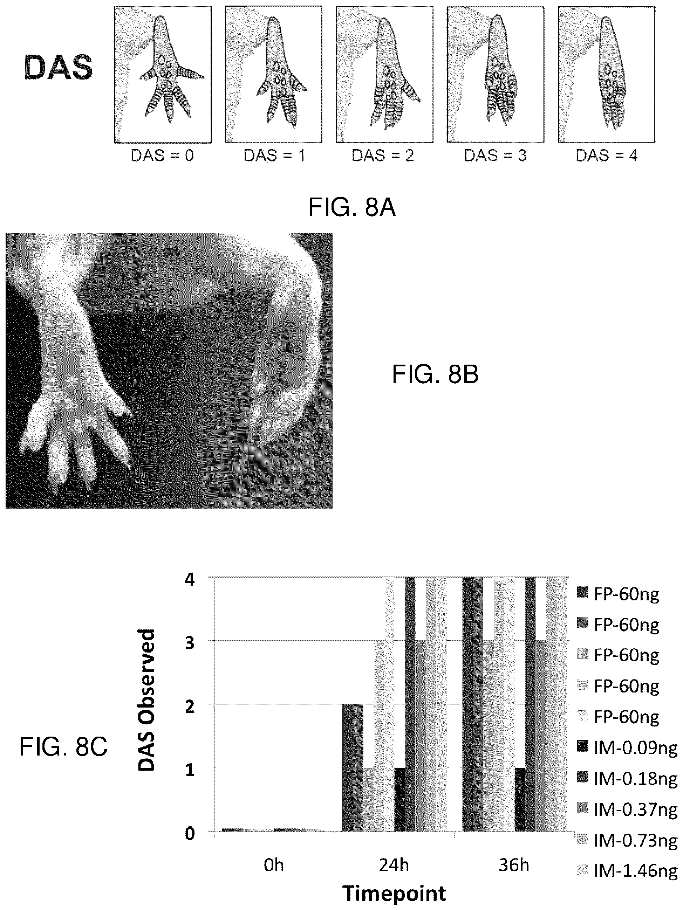

Actual delivery of BTA was measured using the Digital Abduction Scale (DAS). To provide some context, FIG. 8A illustrates a typical DAS scoring metric for mouse paws. FIG. 8B depicts a photograph of a mouse with a DAS score of 4 on the right hind limb following Flex-PAD administration of BTA, and a DAS score of 0 on the left hind limb following Flex-PAD administration of the control. Similarly, as illustrated in FIG. 8C, examination 24-36 hours after treatment showed paralytic effects on digital abduction, with all subjects showing DAS values of "3-4" on the right BTA-treated side. No paralysis (DAS = 0) was observed on control paws with Flex-PADs not containing BTA.

To determine a comparable effective dose in intramuscular (IM) injection of the same BTA solution diluted in phosphate-buffered saline, a dose response study was conducted, administering 90, 180, 360, 720, and 1440 pg BTA, respectively, by 20 µL direct IM injections into gastrocnemius muscle under anesthesia. By 24 h, mice receiving the four highest doses showed DAS values of 3-4, while the lowest dose, 90 pg, produced a DAS=1 value. Representative data of treated subjects in both the Flex-PAD and the IM dose-response experiments are presented in FIG. 8C. It is noted that Flex-PAD DAS scores (FP1-FP5) are presented in sequential order at each time point shown in FIG. 8C followed by IM DAS scores (IM1-IM5), also presented in sequential order at each time point. Saline treated controls on opposite legs of each subject remained at DAS=0 for the duration of the experiment.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the appended claims.

In addition, the following describes additional embodiments:
Item 1: A therapeutic toxin dosage form for treating a disease, disorder, or condition in a subject, comprising:
   a microneedle array, comprising a base portion having microneedles projecting from a surface thereof; and
   a therapeutically effective amount of a therapeutic toxin loaded to the microneedle array for administration to the subject.
Item 2: The therapeutic toxin dosage form of item 1, wherein the microneedles are compositionally homogenous with the base portion.
Item 3: The therapeutic toxin dosage form of item 1, wherein the microneedles comprise a plurality of longitudinal channels.
Item 4: The therapeutic toxin dosage form of item 1, wherein the microneedles are made of a biodegradable/bioabsorbable material.
Item 5: The therapeutic toxin dosage form of item 4, wherein the biodegradable/bioabsorbable material is a member selected from the group consisting of: polyvinyl alcohol, polyvinylpyrrolidone, carbomers, polyacrylic acid, polyoxyethylene/polyoxypropylene copolymers, other copolymers, albumins, casein, zein, collagen, other proteins, glucose, sucrose, maltose, trehalose, amylose, dextrose, fructose, mannose, galactose, other sugars, erythritol, threitol, arabitol, xylitol, ribitol, mannitol, sorbitol, galactitol, fucitol, iditol. inositol, volemitol, isomalt, maltitol, lactitol, maltotriitol, maltotetraitol, polyglycitol, other sugar alcohols, chondroitin and/or other glycosaminoglycans, inulin, starches, acacia gum, agar, carboxymethyl cellulose, methyl cellulose, ethyl cellulose, alginates, carrageenan, cassia gums, cellulose gums, chitin, chitosan, curdlan, gelatin, dextran, fibrin, fulcelleran, gellan gum, ghatti gum, guar gum, tragacanth, karaya gum, locust bean gum, pectin, starch, tara gum, xanthan gum, and other polysaccharides, and functionalized derivatives of any of the above, copolymers thereof, or mixtures thereof.
Item 6: The therapeutic toxin dosage form of item 1, wherein the microneedles are configured to be left in a skin surface of the subject to provide immediate delivery of botulinum toxin, sustained delivery of botulinum toxin even after removal of the base portion, or a combination thereof.
Item 7: The therapeutic toxin dosage form of item 1, wherein the microneedles have a length of from about 10 µm to about 10,000 µm.
Item 8: The therapeutic toxin dosage form of item 1, wherein the microneedles are trimmed to have sharpened tips.
Item 9: The therapeutic toxin dosage form of item 1, wherein the microneedle array has from 1 microneedle to 25,000,000 microneedles.
Item 10: The therapeutic toxin dosage form of item 1, wherein the microneedles are spaced on the base portion at a density of from about 1 microneedle per square centimeter (cm²) to about 2500 microneedles per cm².
Item 11: The therapeutic toxin dosage form of item 1, wherein the therapeutic toxin is included in the microneedles.
Item 12: The therapeutic toxin dosage form of item 1, wherein the therapeutic toxin is localized to a tip of the microneedles.
Item 13: The therapeutic toxin dosage form of item 1, wherein the therapeutic toxin is included in the base portion.
Item 14: The therapeutic toxin dosage form of item 1, wherein the therapeutically effective amount is an amount from about 0.001 units to about 3,000,000 units.
Item 15: The therapeutic toxin dosage form of item 1, wherein the therapeutic toxin is botulinum toxin.
Item 16: The therapeutic toxin dosage form of item 15, wherein botulinum toxin is a member selected from the group consisting of: botulinum toxin A, botulinum toxin B, botulinum toxin C1, botulinum toxin C2, botulinum toxin D, botulinum toxin E, botulinum toxin F, botulinum toxin G, synaptosomal-associated protein 25 (SNAP-25) cleaving agents, and combinations thereof.
Item 17: The therapeutic toxin dosage form of item 15, wherein botulinum toxin comprises botulinum toxin A.
Item 18: The therapeutic toxin dosage form of item 15, wherein botulinum toxin comprises botulinum toxin B.
Item 19: The therapeutic toxin dosage form of item 1, further comprising a second active agent.
Item 20: A method of treating a disease, disorder, or condition in a subject, comprising:
   administering a therapeutically effective amount of a therapeutic toxin to the subject via a microneedle array, the mirconeedle array comprising a base portion having microneedles projecting from a surface thereof.
Item 21: The method of item 20, wherein the disease, disorder, or condition comprises pachyonychia congenita, hyperhidrosis, genodermatosis, epidermolysis bullosa, spastic disorders, facial tics, rosacea, plaque psoriasis, inverse psoriasis, Frey's syndrome, diabetic neuropathy, headaches, skin wrinkles, or a combination thereof.
Item 22: The method of item 20, wherein the subject is a human.
Item 23: The method of item 20, wherein administering comprises applying the microneedle array to a skin surface of the subject for a period of from about 5 seconds to about 24 hours.
Item 24: The method of item 20, wherein administering comprises removing the base portion from a skin surface of the subject while leaving the microneedles embedded in the skin.
Item 25: The method of item 20, wherein the therapeutically effective amount is administered in an effective dosage regimen.
Item 26: The method of item 25, wherein the effective dosage regimen comprises administering botulinum toxin to a subject from 1 to 100 times per day for a period of from 5 seconds to 24 hours.
Item 27: The method of item 25, wherein the effective dosage regimen provides from 0.001 units to 3,000,000 units the therapeutic toxin over a period of 24 hours.
Item 28: The method of item 25, wherein the therapeutically effective amount provides a reduction in sweat production at a site of treatment as compared to sweat production without treatment.
Item 29: The method of item 25, wherein the therapeutically effective amount produces a DAS of greater than or equal to 1 within 36 hours of administration.
Item 30: The method of item 20, wherein the therapeutically effective amount provides a reduction in the symptom of pain associated with the disease, disorder, or condition as compared to pain without treatment.

## Claims

1. A therapeutic toxin dosage form for treating a disease, disorder, or condition in a subject, comprising:
a microneedle array, comprising a base portion having microneedles projecting from a surface thereof; and
a therapeutically effective amount of a therapeutic toxin loaded to the microneedle array for administration to the subject.

2. The therapeutic toxin dosage form of claim 1,
wherein the microneedles are compositionally homogenous with the base portion;
wherein the microneedles comprise a plurality of longitudinal channels;
wherein the microneedles are configured to be left in a skin surface of the subject to provide immediate delivery of botulinum toxin, sustained delivery of botulinum toxin even after removal of the base portion, or a combination thereof; wherein the microneedles have a length of from about 10 µm to about 10,000 µm;
wherein the microneedles are trimmed to have sharpened tips;
wherein the microneedle array has from 1 microneedle to 25,000,000 microneedles; or
wherein the microneedles are spaced on the base portion at a density of from about 1 microneedle per square centimeter (cm²) to about 2500 microneedles per cm².

3. The therapeutic toxin dosage form of claim 1, wherein the microneedles are made of a biodegradable/bioabsorbable material, in particular, wherein the biodegradable/bioabsorbable material is a member selected from the group consisting of: polyvinyl alcohol, polyvinylpyrrolidone, carbomers, polyacrylic acid, polyoxyethylene/polyoxypropylene copolymers, other copolymers, albumins, casein, zein, collagen, other proteins, glucose, sucrose, maltose, trehalose, amylose, dextrose, fructose, mannose, galactose, other sugars, erythritol, threitol, arabitol, xylitol, ribitol, mannitol, sorbitol, galactitol, fucitol, iditol. inositol, volemitol, isomalt, maltitol, lactitol, maltotriitol, maltotetraitol, polyglycitol, other sugar alcohols, chondroitin and/or other glycosaminoglycans, inulin, starches, acacia gum, agar, carboxymethyl cellulose, methyl cellulose, ethyl cellulose, alginates, carrageenan, cassia gums, cellulose gums, chitin, chitosan, curdlan, gelatin, dextran, fibrin, fulcelleran, gellan gum, ghatti gum, guar gum, tragacanth, karaya gum, locust bean gum, pectin, starch, tara gum, xanthan gum, and other polysaccharides, and functionalized derivatives of any of the above, copolymers thereof, or mixtures thereof.

4. The therapeutic toxin dosage form of claim 1,
wherein the therapeutic toxin is included in the microneedles;
wherein the therapeutic toxin is localized to a tip of the microneedles; or
wherein the therapeutic toxin is included in the base portion.

5. The therapeutic toxin dosage form of claim 1, wherein the therapeutically effective amount is an amount from about 0.001 units to about 3,000,000 units.

6. The therapeutic toxin dosage form of claim 1,
wherein the therapeutic toxin is botulinum toxin; in particular, wherein botulinum toxin is a member selected from the group consisting of: botulinum toxin A, botulinum toxin B, botulinum toxin C1, botulinum toxin C2, botulinum toxin D, botulinum toxin E, botulinum toxin F, botulinum toxin G, synaptosomal-associated protein 25 (SNAP-25) cleaving agents, and combinations thereof;
wherein botulinum toxin comprises botulinum toxin A; or
wherein botulinum toxin comprises botulinum toxin B.

7. The therapeutic toxin dosage form of claim 1, further comprising a second active agent.

8. A therapeutic toxin for use in a treatment of a disease, disorder, or condition in a subject, wherein the therapeutic toxin is administered in a therapeutic toxin dosage form of anyone of claims 1 to 7.

9. The therapeutic toxin for use according to claim 8, wherein the disease, disorder, or condition comprises pachyonychia congenita, hyperhidrosis, genodermatosis, epidermolysis bullosa, spastic disorders, facial tics, rosacea, plaque psoriasis, inverse psoriasis, Frey's syndrome, diabetic neuropathy, headaches, skin wrinkles, or a combination thereof.

10. The therapeutic toxin for use according to claim 8, wherein the subject is a human.

11. The therapeutic toxin for use according to claim 8,
wherein administering comprises applying the microneedle array to a skin surface of the subject for a period of from about 5 seconds to about 24 hours; or
wherein administering comprises removing the base portion from a skin surface of the subject while leaving the microneedles embedded in the skin.

12. The therapeutic toxin for use according to claim 8, wherein the therapeutically effective amount is administered in an effective dosage regimen.

13. The therapeutic toxin for use according to claim 12,
wherein the effective dosage regimen comprises administering botulinum toxin to a subject from 1 to 100 times per day for a period of from 5 seconds to 24 hours; or
wherein the effective dosage regimen provides from 0.001 units to 3,000,000 units the therapeutic toxin over a period of 24 hours.

14. The therapeutic toxin for use according to claim 12,
wherein the therapeutically effective amount provides a reduction in sweat production at a site of treatment as compared to sweat production without treatment; or
wherein the therapeutically effective amount produces a DAS of greater than or equal to 1 within 36 hours of administration.

15. The therapeutic toxin for use according to claim 8, wherein the therapeutically effective amount provides a reduction in the symptom of pain associated with the disease, disorder, or condition as compared to pain without treatment.
